# EUROPÄISCHE PATENTSCHRIFT

(11) **EP 2 427 133 B1**
(45) Veröffentlichungstag und Bekanntmachung des Hinweises auf die Patenterteilung: **03.07.2013**
(21) Anmeldenummer: 10715802.4
(22) Anmeldetag: 20.04.2010
(51) Int. Cl.: A61B 19/08, A61B 19/00

(54) **STERILES ABDECKSYSTEM ZUM STERILEN VERKLEIDEN EINES MEDIZINTECHNISCHEN ROBOTERARMS SOWIE VERFAHREN ZUM STERILEN VERKLEIDEN EINES MEDIZINTECHNISCHEN ROBOTERARMS**
STERILE COVER SYSTEM FOR STERILELY COVERING A MEDICAL TECHNICAL ROBOT ARM AND METHOD FOR STERILELY COVERING A MEDICAL TECHNICAL ROBOT ARM
SYSTÈME DE RECOUVREMENT STÉRILE POUR HABILLAGE STÉRILE D'UN BRAS DE ROBOT UTILISÉ EN TECHNIQUE MÉDICALE, ET PROCÉDÉ D'HABILLAGE STÉRILE D'UN BRAS DE ROBOT UTILISÉ EN TECHNIQUE MÉDICALE

(30) Priorität: 05.05.2009 DE 102009019695
(43) Veröffentlichungstag der Anmeldung: 14.03.2012
(73) Patentinhaber: Deutsches Zentrum für Luft- und Raumfahrt e.V., 51147 Köln (DE)
(72) Erfinder: HAGN, Ulrich, 82396 Pähl (DE)
(74) Vertreter: von Kreisler Selting Werner
(86) Internationale Anmeldenummer: PCT/EP2010/055193
(87) Internationale Veröffentlichungsnummer: WO 2010/127940

(56) Entgegenhaltungen:
- WO-A1-92/20295
- GB-A- 2 324 469
- US-A- 5 790 307
- US-A1- 2002 091 374
- US-A1- 2004 049 205

## Beschreibung

Die Erfindung betrifft ein steriles Abdecksystem zum sterilen Verkleiden eines medizintechnischen Roboterarms nach dem Oberbegriff des Anspruchs 1 sowie ein Verfahren zum sterilen Verkleiden eines medizintechnischen Roboterarms.

In der Chirurgie müssen die Oberflächen von Instrumenten, die mit Patienten oder dem Patienteninneren in Kontakt treten, steril, also frei von lebenden Mikroorganismen, sein. Zur Sterilisierung von Geräten gibt es verschiedene Verfahren. Derartige Verfahren sind beispielsweise Dampfsterilisationen, Heißluftsterilisationen, fraktionierte Sterilisation, chemische Sterilisation, Strahlensterilisation oder Plasmasterilisation. Die Sterilisation findet zumeist außerhalb des Operationsbereichs statt, so dass die Geräte steril in diesen verbracht werden. Einige Geräte können jedoch aufgrund ihres Aufbaus oder ihrer Größe nicht sterilisiert werden. Beispielsweise weisen die heutzutage häufig in der Chirurgie verwendeten Medizinroboter Elektronikkomponenten auf, die bei den herkömmlichen Sterilisationsverfahren beschädigt werden können.

Um zu vermeiden, dass nicht sterile Oberflächen der Medizinroboter die sterilisierten Geräte oder Instrumente kontaminieren, werden Medizinroboter üblicherweise Vorort steril verpackt.

Als sterile Verpackung für die Geräte, wie beispielsweise Medizinroboter, dienen meist sterile Verpackungsmaterialien wie Folien, die auch als Drape bezeichnet werden. Im Allgemeinen handelt es sich bei einer Drape um einen sterilisierten Folienschlauch, welcher über das technische Gerät gezogen und fixiert wird. Dabei ist es besonders wichtig, dass während des Anlegens der Drape diese nicht beschädigt und die der sterilen Seite des Operationsfeldes zugewandte sterile Oberfläche der Drape nicht kontaminiert wird.

Im Bereich der endoskopischen robotergestützten Chirurgie kommen endoskopische Instrumente zum Einsatz, die von einem Roboterarm geführt werden. Derartige Instrumente haben einen stabförmigen Teil, der ein funktionales Ende, wie beispielsweise eine Zange oder eine Schere, aufweist. Das funktionale Ende kommt dabei direkt mit dem Patienteninneren in Kontakt und muss daher steril sein. Bei einem Robotersystem, wie es beispielsweise in DE 10 2004 054 866 B3 beschrieben ist, koppelt das Instrument derart mit dem Robotersystem, dass das Instrument mit dem funktionellen Ende voran durch eine Hohlwelle des Roboters geschoben wird. Ein Kontakt der Innenwandung der Hohlwelle mit dem funktionalen Ende des Instrumentes kann daher zur Kontamination des funktionalen Endes führen.

Das Dokument DI (US 5790307), das die Basis des Oberbegriffs von Anspruch 1 formt, offenbart ein Steriles Abdecksystem zwi sterilen Verkleiden eines medizintechnischen Roboterarms mit einem, eine Aufnahmebohrung mit Innenwandung aufweisenden Anschusselement für Endoskopische Geräte, mit einer backförmigen Drupe zum Verhüllen des Anschlusselementes.

Zur Sterilisation einer Hohlwelle eines Roboterarmes ist jedoch nur mit sehr hohem Aufwand oder nicht möglich. Die bekannten sterilen Verpackungen in Form von Drapes können zwar den Roboterarm steril verkleiden, jedoch führt die Auskleidung der Hohlwelle zu einem Problem, da ein durch die Hohlwelle durchgezogenes Drape Falten schlagen kann und die Folienflächen, die im späteren sterilen Bereich liegen, beim Durchschieben kontaminiert werden können.

Es ist daher die Aufgabe der vorliegenden Erfindung, ein steriles Abdecksystem zum sterilen Verkleiden eines medizintechnischen Roboterarms mit einem eine Aufnahmebohrung aufweisenden Anschlusselement für endoskopische Geräte zu schaffen, bei dem unter Vermeidung der zuvor genannten Nachteile ein zuverlässiges steriles Verkleiden, insbesondere von dem Anschlusselement, möglich ist.

Eine weitere Aufgabe der Erfindung liegt ferner darin, ein Verfahren zum zuverlässigen sterilen Verkleiden eines medizintechnischen Roboterarms mit einem eine Aufnahmebohrung aufweisenden Anschlusselement für endoskopische Geräte zu schaffen.

Die Aufgaben der Erfindung werden durch die Merkmale der Ansprüche 1 und 16 gelöst.

Das erfindungsgemäße sterile Abdecksystem zum sterilen Verkleiden eines medizinischen Roboterarms mit einem eine Aufnahmebohrung mit Innenwand aufweisenden Anschlusselement für endoskopische Geräte weist eine sackförmige Drape zum Umhüllen des Anschlusselementes und einen an die Aufnahmebohrung angepassten Folienschlauch zur Abdeckung der Innenwandung auf. An einem ersten Ende des Folienschlauches ist ein abnehmbares Führungselement befestigt, das an die Aufnahmebohrung angepasst ist und im Ausgangszustand des sterilen Abdecksystems in den Folienschlauch hineinragt. Der Folienschlauch ist mit dem Führungselement in die Aufnahmebohrung einführ- und durchführbar, derart, dass eine im Ausgangszustand des sterilen Abdecksystems nach innen gewandte Oberfläche des Folienschlauches im durch die Aufnahmebohrung durchgeführten Zustand des Folienschlauches der Innenwandung der Aufnahmebohrung zugewandt ist.

Mit Hilfe des Führungselementes wird ein exaktes Führen des Folienschlauches durch die Aufnahmebohrung des Anschlusselementes für endoskopische Geräte ermöglicht, so dass Falten des Folienschlauches vermieden werden. Mit Hilfe des Führungselementes, das im Ausgangszustand in den Folienschlauch hineinragt, ist es möglich, den Folienschlauch derart in die Aufnahmebohrung ein- und durchzuführen, dass der Folienschlauch während des Ein- bzw. Durchführens umgestülpt wird. Bei dem Ein- und Durchführen des Folienschlauches durch die Aufnahmebohrung führt die Oberfläche des Folienschlauches keine Gleitbewegung, sondern eine Abwälzbewegung aus. Auf diese Weise wird das Kontaminieren weiterer Oberflächen des Folienschlauches verhindert und gewährleistet, dass die im durchgeführten Zustand des Folienschlauches nach innen, von der Aufnahmebohrung weg gerichtete Oberfläche, die beim späteren Durchführen eines endoskopischen Gerätes durch die Aufnahmebohrung mit dem endoskopischen Gerät in Berührung kommen kann, zuvor keinen Kontakt mit der Innenwandung des Anschlusselementes oder einem anderen Teil des Roboterarmes bekommen konnte. Ein zuverlässiges steriles Abdecken der Innenwandung der Aufnahmebohrung und des Anschlusselementes für endoskopische Geräte wird somit gewährleistet.

Darüber hinaus kann der Folienschlauch mit Hilfe des Führungselementes in vorteilhafter Weise gehandhabt bzw. geführt werden.

Es kann vorgesehen sein, dass das Führungselement stabförmig ausgebildet ist, wobei vorzugsweise an einem freien Ende des Führungselementes eine Spitze, die vorzugsweise zum Durchstechen der Drape geeignet ist, angeordnet ist. Durch die stabförmige Ausbildung des Führungselementes ist das Führungselement in vorteilhafter Weise durch die Aufnahmebohrung des Anschlusselementes durchführbar. Die Spitze an einem freien Ende des Führungselementes der Drape ermöglicht beispielsweise, dass in dem sterilen Abdecksystem herkömmliche Drapes verwendet werden können, da vor dem Durchführen des Folienschlauches durch die Aufnahmebohrung mit Hilfe des Führungselementes die Drape an entsprechender Stelle durchstochen werden kann, um den Folienschlauch in das Innere der Drape und in die Aufnahmebohrung einzuführen. Mit der Spitze kann ferner nach Durchführung des Folienschlauches durch die Aufnahmebohrung das an dem anderen Ende des Anschlusselementes anliegende Drape durchstochen werden. Darüber hinaus ermöglicht die Spitze an einem freien Ende des Führungselementes ein vorteilhaftes Einführen des Führungselementes in die Aufnahmebohrung.

In einem besonders bevorzugten Ausführungsbeispiel ist vorgesehen, dass die sackförmige Drape mindestens eine an das Führungselement und/oder die Aufnahmebohrung angepasste Öffnung mit umlaufender Kante aufweist, durch die das Führungselement und der Folienschlauch zumindest durchführbar sind. Durch das Vorsehen einer ersten Öffnung an der sackförmigen Drape wird das Durchführen des Führungselementes und des Folienschlauches durch die Aufnahmebohrung vereinfacht, da nicht mit Hilfe des Führungselementes eine Öffnung in der sackförmigen Drape gebildet werden muss.

Darüber hinaus kann mit Hilfe der ersten Öffnung die Drape exakt an dem Anschlusselement positioniert werden, indem die Öffnung im Bereich der Aufnahmebohrung angeordnet wird.

Dabei kann vorgesehen sein, dass der Folienschlauch mit einem zweiten Ende an der umlaufenden Kante befestigbar oder befestigt ist. Auf diese Weise wird das Durchführen des Folienschlauches durch die Aufnahmebohrung vereinfacht, da durch die Befestigung des zweiten Endes des Folienschlauches an der umlaufenden Kante der ersten Öffnung die Gefahr, dass der Folienschlauch beim Durchführen durch die Aufnahmebohrung durch die Aufnahmebohrung durchrutscht, vermieden wird.

In einem Ausführungsbeispiel der Erfindung ist vorgesehen, dass eine erste Platte mit Durchgangsbohrung an der Drape angeordnet ist, wobei das Durchgangsloch die erste Öffnung bildet. Mit Hilfe einer ersten Platte mit Durchgangsloch kann die Drape in vorteilhafter Weise an dem Anschlusselement positioniert werden. Darüber hinaus ermöglicht die erste Platte in vorteilhafter Weise eine Befestigung des Folienschlauches an der ersten Öffnung.

Es kann vorgesehen sein, dass der Folienschlauch mit dem zweiten Ende fest mit der Platte verbunden ist. Auf diese Weise wird nicht nur gewährleistet, dass der Folienschlauch beim Durchführen durch die Aufnahmeöffnung nicht durchrutschen kann, sondern Folienschlauch und Drape können darüber hinaus als eine Einheit an dem Anschlusselement angebracht werden.

In einem alternativen Ausführungsbeispiel ist vorgesehen, dass der Folienschlauch mit dem zweiten Ende mit der Platte mechanisch oder stoffschlüssig verbindbar ist. Der Folienschlauch wird somit vor dem Ein- und Durchführen durch die Aufnahmeöffnung über die erste Platte mit der Drape verbunden, so dass ein Durchrutschen des Folienschlauches verhindert wird.

In einem bevorzugten Ausführungsbeispiel ist vorgesehen, dass der Folienschlauch aus elastischem Material, vorzugsweise Silikon, besteht. Das Vorsehen eines Folienschlauches aus elastischem Material verhindert, dass der Folienschlauch beim Durchführen in die Aufnahmebohrung Falten schlagen kann. Da Silikon in vorteilhafter Weise sterilisierbar ist, hat sich dieses Material als besonders vorteilhaft für einen erfindungsgemäßen Folienschlauch herausgestellt.

Die erfindungsgemäße Drape kann mindestens eine an das Führungselement und/oder die Aufnahmebohrung angepasste zweite Öffnung mit umlaufender Kante aufweisen, durch die das Führungselement und der Folienschlauch zumindest teilweise durchführbar sind. Mit Hilfe der zweiten Öffnung ist die Drape in vorteilhafter Weise an dem Anschlusselement positionierbar und das Führungselement und das erste Ende des Folienschlauchs sind nach dem Durchführen durch die Aufnahmeöffnung durch die Drape durchführbar.

Dabei kann vorgesehen sein, dass der Folienschlauch mit dem ersten Ende an der umlaufenden Kante der zweiten Öffnung befestigbar ist. Dadurch wird ermöglicht, dass nach dem Durchführen des Folienschlauches durch die Aufnahmebohrung der Folienschlauch und die sackförmige Drape eine fest verbundene Einheit bilden, so dass alle der Umgebung zugewandten Oberflächen des Anschlusselementes entweder von der Drape oder dem Folienschlauch abgedeckt sind.

An der Drape kann eine zweite Platte mit Durchgangsloch angeordnet sein, wobei das Durchgangsloch der zweiten Platte die zweite Öffnung bildet. Mit Hilfe der Platte mit Durchgangsloch ist die Drape in vorteilhafter Weise an dem Anschlusselement positionierbar.

Darüber hinaus ist die Befestigung des ersten Endes des Folienschlauches an der umlaufenden Kante der zweiten Öffnung durch die zweite Platte in vorteilhafter Weise möglich.

Es kann vorgesehen sein, dass der Folienschlauch mit dem ersten Ende mit der zweiten Platte mechanisch oder stoffschlüssig verbindbar ist.

In einem besonders bevorzugten Ausführungsbeispiel ist vorgesehen, dass die erste und/oder die zweite Platte an dem Anschlusselement fixierbar ist, vorzugsweise mechanisch, magnetisch oder stoffschlüssig. Auf diese Weise wird nicht nur eine genauere Positionierung der Drape an dem Anschlusselement ermöglicht, sondern die Drape kann gleichzeitig mit Hilfe der ersten und/oder der zweiten Platte an dem Anschlusselement befestigt werden. Darüber hinaus wird verhindert, dass beim Durchführen des Folienschlauches durch die Aufnahmebohrung des Anschlusselementes die Drape verrutschen kann.

In einem Ausführungsbeispiel des erfindungsgemäßen sterilen Abdecksystems ist vorgesehen, dass die erste und/oder die zweite Platte ein Klemmelement zur Befestigung des jeweiligen Folienschlauches aufweist, wobei das Klemmelement vorzugsweise plattenförmig ausgebildet ist. Die mechanische Befestigung des ersten Endes des Folienschlauches an der zweiten Platte und/oder des zweiten Endes des Folienschlauches an der ersten Platte über ein Klemmelement ermöglicht eine einfache und schnelle Befestigung der jeweiligen Enden des Folienschlauches an einer der Platten.

Die erste und/oder die zweite Platte und/oder das Klemmelement können unterschiedliche Formen aufweisen. Beispielsweise können diese Elemente ringförmig ausgestaltet sein.

Dabei kann das Klemmelement jeweils über eine Schnapp- und/oder Schraubverbindung mit der ersten und/oder zweiten Platte zum Erzeugen einer Klemmkraft verwendbar sein.

Die Erfindung sieht ferner ein Verfahren zum sterilen Verkleiden eines medizintechnischen Roboterarms mit einem eine Aufnahmebohrung mit Innenwandung aufweisenden Anschlusselement für endoskopische Geräte mit folgenden Schritten vor:
a) Umhüllen des Roboterarms mit einer sackförmigen Drape,
b) Einführen eines Folienschlauches in eine erste Öffnung in der sackförmigen Drape und in die Aufnahmebohrung mit Hilfe eines an einem ersten Ende des Folienschlauches befestigten Führungselementes,
c) Durchführen des Folienschlauches durch die Aufnahmebohrung mit Hilfe des Führungselementes derart, dass die im Ausgangszustand des Folienschlauches nach innen gewandte Oberfläche des Folienschlauches im durch die Aufnahmebohrung durchgeführten Zustand des Folienschlauches der Innenwandung der Aufnahmebohrung zugewandt ist,
d) Durchführen des Folienschlauches durch eine zweite Öffnung in der sackförmigen Drape,
e) Lösen des Führungselementes von dem ersten Ende des Folienschlauches,
f) Befestigen des ersten Endes des Folienschlauches an der sackförmigen Drape im Bereich der zweiten Öffnung.

Das erfindungsgemäße Verfahren ermöglicht ein zuverlässiges steriles Verkleiden eines medizintechnischen Roboterarms mit einem eine Aufnahmebohrung aufweisenden Anschlusselement für endoskopische Geräte, da bei dem Durchführen des Folienschlauches durch die Aufnahmebohrung gewährleistet wird, dass die später mit dem endoskopischen Gerät in Berührung kommenden Oberflächen des Folienschlauches nicht durch unsterile Oberflächen des Anschlusselementes kontaminiert werden können. Dies wird gewährleistet, indem der Folienschlauch beim Durchführen durch die Aufnahmebohrung umgestülpt wird. Dabei führt die Oberfläche des Folienschlauches auf der Oberfläche der Aufnahmebohrung keine Gleitbewegung, sondern eine Abwälzbewegung durch, wodurch eine Kontaminierung anderer Bereiche des Folienschlauches vermieden wird.

Bei dem erfindungsgemäßen Verfahren kann vorgesehen sein, dass im Ausgangszustand der Drape die erste Öffnung in der sackförmigen Drape angeordnet ist, wobei die erste Öffnung an das Führungselement oder die Aufnahmebohrung angepasst ist.

Die Anordnung der ersten Öffnung in der sackförmigen Drape ermöglicht die Positionierung der Drape an dem Anschlusselement in vorteilhafter Weise.

Dabei kann vorgesehen sein, dass im Ausgangszustand der Drape der Folienschlauch mit dem zweiten Ende im Bereich der ersten Öffnung der sackförmigen Drape befestigt ist.

Dadurch, dass im Ausgangszustand der Drape der Folienschlauch mit dem zweiten Ende bereits an der sackförmigen Drape befestigt ist, wird verhindert, dass beim Durchführen des Folienschlauches durch die Aufnahmebohrung der Folienschlauch durch die Aufnahmebohrung durchrutschten kann. Darüber hinaus wird ein weiterer Verfahrensschritt des Befestigens des zweiten Endes an dem Bereich der ersten Öffnung vermieden.

Es kann vorgesehen sein, dass im Ausgangszustand der Drape die zweite Öffnung an der sackförmigen Drape angeordnet ist, wobei die zweite Öffnung an das Führungselement und/oder die Aufnahmebohrung angepasst ist.

In einem alternativen Ausführungsbeispiel kann vorgesehen sein, dass zwischen Schritt a) und b) folgender Zwischenschritt durchgeführt wird:
a1) Durchstoßen der sackförmigen Drape mit Hilfe des Führungselementes zum Bilden der ersten Öffnung in der sackförmigen Drape.

Auch kann vorgesehen, dass zwischen Schritt c) und d) folgender Zwischenschritt durchgeführt wird:
c1) Durchstoßen der sackförmigen Drape mit Hilfe des Führungselementes zum Bilden der zweiten Öffnung in der sackförmigen Drape.

Durch das Vorsehen der zusätzlichen Zwischenschritte a1) und/oder c1), wodurch die erste bzw. zweite Öffnung in der sackförmigen Drape durch Durchstoßen der sackförmigen Drape mit Hilfe des Führungselementes gebildet werden, kann die in dem erfindungsgemäßen Verfahren verwendete sackförmige Drape an unterschiedlichen Anschlusselementen verwendet werden. Erst nach Umhüllen des Roboterarms mit einer erfindungsgemäßen sackförmigen Drape werden mit Hilfe dieser beiden Verfahrensschritte die notwendigen Öffnungen an den entsprechenden Positionen in der Drape gebildet.

Das erfindungsgemäße Verfahren kann ferner zwischen Schritt a) und b) folgenden weiteren Zwischenschritt aufweisen:
a2) Befestigen eines zweiten Endes des Folienschlauches an der sackförmigen Drape im Bereich der ersten Öffnung.

Das Vorsehen dieses Schrittes verhindert, dass bei einer Drape, bei der der Folienschlauch im Ausgangszustand nicht mit der Drape verbunden ist, der Folienschlauch beim Durchführen des Folienschlauches durch die Aufnahmebohrung durch die Aufnahmebohrung durchrutschen kann.

Im Folgenden wird unter Bezugnahme auf die nachfolgenden Zeichnungen die Erfindung näher erläutert. Es zeigen:
- Fig. 1: eine Schnittdarstellung eines ersten Ausführungsbeispiels eines erfindungsgemäßen sterilen Abdecksystems,
- Fign. 2-4: eine schematische Schnittdarstellungen eines zweiten Ausführungsbeispiels eines erfindungsgemäßen sterilen Abdecksystems in verschiedenen Positionen beim Durchführen eines erfindungsgemäßen Verfahrens,
- Fig. 5: eine schematische Schnittdarstellung eines dritten Ausführungsbeispiels eines erfindungsgemäßen sterilen Abdecksystems.

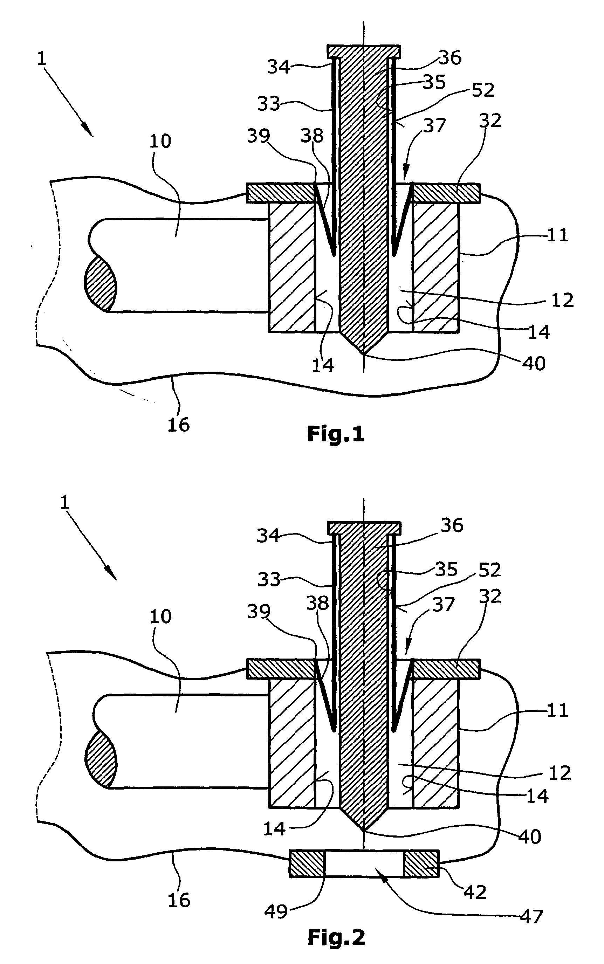

In Fig. 1 ist eine schematische Schnittdarstellung eines ersten Ausführungsbeispiels eines erfindungsgemäßen Abdecksystems 1 dargestellt. Das erfindungsgemäße Abdecksystem 1 ist zum sterilen Verkleiden eines medizintechnischen Roboterarms 10 mit einem eine Aufnahmebohrung 12 mit Innenwandung 14 aufweisenden Anschlusselement 11 für endoskopische Geräte ausgebildet.

Eine sackförmige Drape 16 umhüllt das Anschlusselement 11 sowie einen Teil oder den gesamten Roboterarm 10.

In die Drape 16 ist eine erste Platte 32 mit Durchgangsloch eingelassen, wobei das Durchgangsloch eine erste Öffnung 37 in der Drape 16 bildet.

Mit Hilfe der ersten Platte 32 ist die sackförmige Drape 16 in vorteilhafter Weise an dem medizintechnischen Roboterarm 10 positionierbar, indem die erste Platte 32 auf dem Anschlusselement 11 aufgelegt wird, derart, dass die erste Öffnung 37 mit der Aufnahmebohrung 12 fluchtet. Dabei kann die sackförmige Drape 16 mit Hilfe der ersten Platte 32 an dem medizintechnischen Roboterarm 10 befestigt werden, indem die erste Platte 32 mechanisch, magnetisch oder stoffschlüssig an dem Anschlusselement 11 befestigt wird.

Das sterile Abdecksystem 1 weist ferner einen an die Aufnahmebohrung 12 angepassten Folienschlauch 33 auf, der zur Abdeckung der Innenwandung 14 der Aufnahmebohrung 12 geeignet ist. An dem Folienschlauch 33 ist an einem ersten Ende 34 des Folienschlauches 33 ein Führungselement 36 abnehmbar befestigt. Unter "abnehmbar befestigt" im Rahmen der Erfindung werden auch mechanische und stoffschlüssige Befestigungen zweier Element inklusive irreversible lösbare Befestigungen, wie beispielsweise Schweißverbindungen, die nur durch Zerschneiden lösbar sind, verstanden.

Der Folienschlauch 33 ist mit einem zweiten Ende 35 an der umlaufende Kante 39 der ersten Öffnung 37 befestigt.

Im Ausgangszustand des sterilen Abdecksystems 1 ragt das Führungselement 36 in den Folienschlauch 33 hinein. Das Führungselement 36 ist derart ausgestaltet, dass mit Hilfe des Führungselementes 36 der Folienschlauch 33 zumindest teilweise in die Aufnahmebohrung einführ- und durchführbar ist. Im Ausgangszustand des sterilen Abdecksystems 1 ist eine nach innen gewandte Oberfläche 35 des Folienschlauches 33 dem Führungselement 36 zugewandt.

Bei der Einführ- und Durchführbewegung des Führungselementes 36 und des Folienschlauches 33 durch die Aufnahmebohrung 12 wird der Folienschlauch 33 umgestülpt, so dass die zuvor nach innen gewandte Oberfläche 35 des Folienschlauches 33 im durch die Aufnahmebohrung 12 durchgeführten Zustand des Folienschlauches 33 der Innenwandung 14 der Aufnahmebohrung 12 zugewandt ist. Dabei führt die Oberfläche 35 des Folienschlauches 33 an der Innenwandung 14 der Aufnahmebohrung 12 eine Abwälzbewegung durch, so dass es nur zu einer geringen oder keinen Gleitbewegung zwischen den Oberflächen kommt, wodurch die Gefahr, dass andere Bereiche oder Oberflächen des Folienschlauches 33 von der Aufnahmebohrung 12 kontaminiert werden, verhindert wird. Insbesondere die Oberfläche 52 des Folienschlauches 33, die im durchgeführten Zustand des Folienschlauches 33 nach innen gewandt ist und direkt ein endoskopisches Instrument berühren kann, kann bei dieser Vorgehensweise nicht mit der unsterilen Innenwandung 14 der Aufnahmebohrung 12 in Kontakt treten und kontaminiert werden.

Das Führungselement 36 ist stabförmig ausgebildet und weist an einem freien Ende eine Spitze 40 auf. Mit Hilfe der Spitze 40 wird nicht nur das Einführen des Führungselementes in die Aufnahmebohrung erleichtert, sondern es ist auch möglich, den sich unterhalb der Aufnahmebohrung 12 befindlichen Teil der Drape zum Bilden einer zweiten Öffnung zu durchstechen, wodurch der Folienschlauch 33 nach Durchführen durch die Aufnahmebohrung 12 teilweise aus der Drape 16 herausgeführt werden kann.

Danach kann das Führungselement 36 von dem Folienschlauch 33 abgenommen werden und das erste Ende 34 des Folienschlauches 33 kann beispielsweise durch Verkleben an der Drape 16 befestigt werden. Auf diese Weise sind sämtliche Oberflächen des Roboterarms 10 mit sterilem Material abgedeckt.

In den Fign. 2 bis 4 ist eine schematische Schnittdarstellung eines zweiten Ausführungsbeispiels eines erfindungsgemäßen sterilen Abdecksystems in verschiedenen Positionen beim Durchführen eines erfindungsgemäßen Verfahrens dargestellt. Das sterile Abdecksystem 1 unterscheidet sich von dem sterilen Abdecksystem der Fig. 1 lediglich darin, dass die Drape 16 gegenüberliegend von der ersten Platte 32 eine zweite Platte 42 mit einer zweiten Öffnung 47 mit umlaufender Kante 49 aufweist.

Die sackförmige Drape 16 des sterilen Abdecksystems 1, an der ein Folienschlauch 33 mit einem an einem ersten Ende 34 befestigten Führungselement 36 angeordnet ist, wird zunächst über einen medizintechnischen Roboterarm 10, der ein Anschlusselement 11 mit Aufnahmebohrung 12 aufweist, gestülpt. In der Drape 16 ist eine erste Platte 32 mit einem Durchgangsloch, das eine erste Öffnung 37 in der sackförmigen Drape bildet, angeordnet.

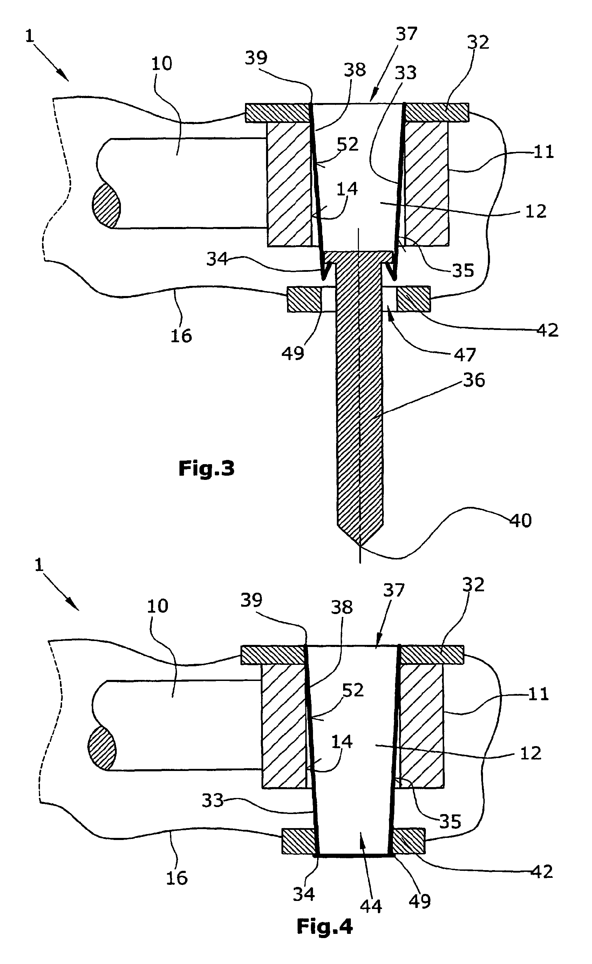

Mit Hilfe der Platte 32 kann die Drape 16 in vorteilhafter Weise auf dem Anschlusselement 11 angeordnet werden, so dass die erste Öffnung 37 mit der Aufnahmebohrung 12 fluchtet. Der Folienschlauch 33 mit dem Führungselement 36 befindet sich dabei außerhalb der Drape 16, wobei das Führungselement 36 in den Folienschlauch 33 hineinragt. Die Drape 16 wird an dem medizintechnischen Roboterarm 10 befestigt. Dazu kann beispielsweise die erste Platte 32 dienen, mit der die Drape 16 magnetisch, mechanisch oder stoffschlüssig, beispielsweise durch Kleben, an dem Anschlusselement 11 des Roboterarms 10 fixiert werden kann. Der Folienschlauch 33 wird mit Hilfe des Führungselementes 36 nun in die Aufnahmebohrung 12 eingeführt und wie aus Fig. 3 ersichtlich ist, durch die Aufnahmebohrung 12 hindurchgeführt.

Da der Folienschlauch 33 mit einem zweiten Ende 38 an der umlaufenden Kante 39 der ersten Öffnung 37 befestigt ist, wird der Folienschlauch 33 bei dem Ein- und Durchführen durch die Aufnahmebohrung 12 umgestülpt, wobei gleichzeitig verhindert wird, dass bei der Ein- und Durchführbewegung durch die Aufnahmebohrung 12 der Folienschlauch 33 durch die Aufnahmebohrung 12 durchrutscht.

Bei der Ein- und Durchführbewegung des Folienschlauches 33 durch die Aufnahmebohrung 12 führt die im Ausgangszustand des Folienschlauches 33 nach innen und dem Führungselement 36 zugewandte Oberfläche 35 eine Abwälzbewegung an der Innenwandung 14 der Aufnahmebohrung 12 durch, so dass es zu keiner oder nur geringer Gleitbewegung zwischen den Oberflächen des Anschlusselementes 11 und dem Folienschlauch 33 kommt. Dadurch wird verhindert, dass die anderen Oberflächen des Folienschlauches 33, außer der im Ausgangszustand nach innen gewandten Oberfläche 35 des Folienschlauches 33, mit Oberflächen des Anschlusselementes 11 in Berührung kommen. Eine Kontaminierung dieser Oberflächen des Folienschlauches 33 wird somit verhindert. Insbesondere die Oberfläche 52 des Folienschlauches 33, die im durchgeführten Zustand des Folienschlauches 33 nach innen gewandt ist und direkt mit einem endoskopischen Instrument in Kontakt treten kann, kann bei dieser Vorgehensweise nicht durch die unsterile Innenwandung 14 der Aufnahmebohrung 12 kontaminiert werden.

Wie aus den Fign. 3 und 4 hervorgeht, wird das Führungselement 36 zusammen mit einem Teil des Folienschlauches 33 nach der Durchführung durch die Aufnahmebohrung 12 durch die zweite Öffnung 47 der zweiten Platte 42 durchgeführt, so dass sich das Führungselement 36 und ein Teil des Folienschlauches 33, das das erste Ende 34 des Folienschlauches 33 umfasst, sich außerhalb der Drape 16 befindet. Das Führungselement 36 wird nun von dem Folienschlauch 33 abgenommen, beispielsweise indem es abgeschnitten wird.

Wie aus Fig. 4 hervorgeht, wird nun das erste Ende 34 des Folienschlauches 33, das nunmehr ein freies Ende ist, an der umlaufenden Kante 49 der zweiten Platte 42 befestigt. Dies kann mechanisch oder stoffschlüssig, beispielsweise durch Kleben, erfolgen.

Durch das Anwenden des erfindungsgemäßen Verfahrens zum sterilen Verkleiden des medizintechnischen Roboterarms 10 sind nunmehr alles Oberflächen, inklusive der Innenwandung der Aufnahmebohrung des Anschlusselementes 11, zuverlässig steril verkleidet. Bei einem Befestigen eines endoskopischen Gerätes an dem Anschlusselement 11 kann das endoskopische Gerät nicht mit dem Anschlusselement 11 in Berührung kommen, so dass es nicht zu einer Kontaminierung des endoskopischen Gerätes kommen kann.

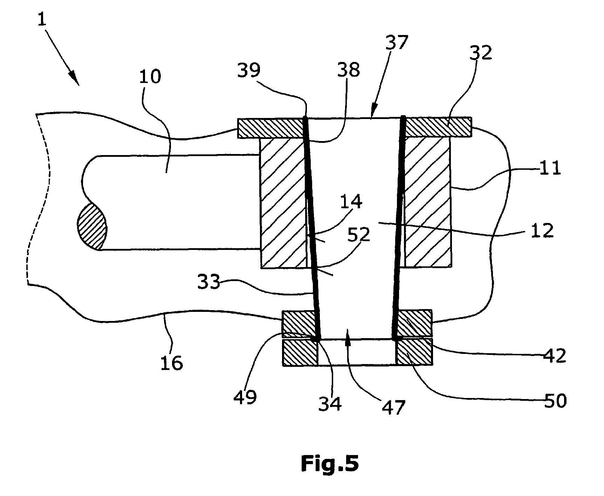

In Fig. 5 ist ein weiteres Ausführungsbeispiel eines erfindungsgemäßen sterilen Abdecksystems 1 in schematischer Schnittdarstellung gezeigt. In Fig. 5 ist das sterile Abdecksystem 1 im an dem Anschlusselement 11 des medizintechnischen Roboterarms 10 angebrachten Zustand gezeigt.

Die Darstellung in Fig. 5 gleicht somit im Wesentlichen der Darstellung des zweiten Ausführungsbeispiels in Fig. 4. Das in Fig. 5 gezeigte Ausführungsbeispiel unterscheidet sich von dem in Fig. 4 dargestellten Ausführungsbeispiel in einem Klemmelement 50, das zur Befestigung des ersten Endes 34 des Folienschlauches 33 an der umlaufenden Kante 49 der zweiten Platte 42 mechanisch mit der zweiten Platte 42 befestigt ist.

Das Klemmelement 50 kann beispielsweise über eine Schnappverbindung oder eine Schraubverbindung mit der zweiten Platte 42 befestigt werden. Auf diese Weise ist eine besonders stabile Befestigung des ersten Endes 34 an der zweiten Öffnung 47 der Drape 16 möglich.

Bei einem erfindungsgemäßen sterilen Abdecksystem 1 kann der Folienschlauch 33 aus einem elastischen Kunststoff, beispielsweise Silikon, gefertigt sein. Die elastische Ausführung des Folienschlauches 33 hat den Vorteil, dass beim Durchführen des Folienschlauches 33 durch die Aufnahmebohrung 12 es nicht oder nur zu geringer Faltenbildung in dem Folienschlauch 33 kommt.

Der Folienschlauch muss nicht zwangsweise bereits an der Drape befestigt sein. Selbstverständlich ist es auch möglich, dass die Drape und der Folienschlauch separate Teile sind, wobei das zweite Ende des Folienschlauches vor dem Durchführen durch die Aufnahmebohrung an der ersten Öffnung in der Drape befestigt wird. Dies kann beispielsweise über ein weiteres Klemmelement erfolgen, das an der ersten Platte befestigt wird.

Die Drape muss nicht zwangsweise Platten mit den entsprechenden Durchgangsbohrungen für die Öffnungen aufweisen, sondern es ist auch möglich, dass entweder die Öffnungen direkt in der Drape, d.h. ohne entsprechende Platten angeordnet sind oder dass die Drape ohne Öffnungen ausgeliefert wird, wobei mit Hilfe des Führungselementes die entsprechenden Öffnungen in die Drape hineingestochen werden können.

Auch ist es möglich, dass die Drape sowohl mit der ersten Platte als auch mit der zweiten Platte an dem Anschlusselement befestigt ist.

Das sterile Abdecksystem ist für jede Art von medizintechnischen Roboterarmen mit einem Anschlusselement für endoskopische Geräte geeignet, das eine Aufnahmebohrung für die endoskopischen Geräte besitzt. Das Anschlusselement mit Aufnahmebohrung kann beispielsweise auch eine Hohlwelle sein.

## Patentansprüche

1. Steriles Abdecksystem (1) zum sterilen Verkleiden eines medizintechnischen Roboterarms (10) mit einem eine Aufnahmebohrung (12) mit Innenwandung (14) aufweisenden Anschlusselement (11) für endoskopische Geräte, mit
einer sackförmigen Drape (16) zum Umhüllen des Anschlusselementes (11),
**gekennzeichnet durch**
einen an die Aufnahmebohrung (12) angepassten Folienschlauch (33) zur Abdeckung der Innenwandung (14) und
ein an einem ersten Ende (34) des Folienschlauches (33) befestigtes, abnehmbares Führungselement (36), das an die Aufnahmebohrung (12) angepasst ist und im Ausgangszustand des sterilen Abdecksystems in den Folienschlauch (33) hineinragt,
wobei der Folienschlauch (33) mit dem Führungselement (36) in die Aufnahmebohrung (12) einführbar und durchführbar ist, derart, dass eine im Ausgangszustand des sterilen Abdecksystems nach innen gewandte Oberfläche (35) des Folienschlauches (33) im **durch** die Aufnahmebohrung (12) durchgeführten Zustand des Folienschlauches (33) der Innenwandung (14) der Aufnahmebohrung (12) zugewandt ist.

2. Steriles Abdecksystem nach Anspruch 1 **dadurch gekennzeichnet, dass** das Führungselement (36) stabförmig ausgebildet ist, wobei vorzugsweise an einem freien Ende des Führungselementes (36) eine Spitze (40), die vorzugsweise zum Durchstechen der Drape geeignet ist, angeordnet ist.

3. Steriles Abdecksystem nach Anspruch 1 oder 2, **dadurch gekennzeichnet, dass** die sackförmige Drape (16) mindestens eine an das Führungselement (36) und/oder die Aufnahmebohrung (12) angepasste erste Öffnung (37) mit umlaufender Kante (39) aufweist, durch die das Führungselement (36) und der Folienschlauch (33) zumindest teilweise durchführbar sind.

4. Steriles Abdecksystem nach Anspruch 3, **dadurch gekennzeichnet, dass** der Folienschlauch (33) mit einem zweiten Ende (35) an der umlaufenden Kante (39) der ersten Öffnung (37) befestigbar oder befestigt ist.

5. Steriles Abdecksystem nach Anspruch 3 oder 4, **dadurch gekennzeichnet, dass** eine erste Platte (32) mit Durchgangsloch an der Drape angeordnet ist, wobei das Durchgangsloch die erste Öffnung (37) bildet.

6. Steriles Abdecksystem nach Anspruch 5, **dadurch gekennzeichnet, dass** der Folienschlauch (33) mit dem zweiten Ende (38) fest mit der ersten Platte (32) verbunden ist.

7. Steriles Abdecksystem nach Anspruch 5, **dadurch gekennzeichnet, dass** der Folienschlauch (33) mit dem zweiten Ende (38) mit der ersten Platte (32) mechanisch oder stoffschlüssig verbindbar ist.

8. Steriles Abdecksystem nach einem der Ansprüche 1 bis 7, **dadurch gekennzeichnet, dass** der Folienschlauch (33) aus elastischem Material, vorzugsweise Silikon, besteht.

9. Steriles Abdecksystem nach einem der Ansprüche 3 bis 8, **dadurch gekennzeichnet, dass** die sackförmige Drape (16) mindestens eine an das Führungselement und/oder die Aufnahmebohrung angepasste zweite Öffnung (47) mit umlaufender Kante (49) aufweist, durch die das Führungselement (36) und der Folienschlauch (33) zumindest teilweise durchführbar sind.

10. Steriles Abdecksystem nach Anspruch 9, **dadurch gekennzeichnet, dass** der Folienschlauch (33) mit dem ersten Ende (34) an der umlaufenden Kante (49) der zweiten Öffnung (47) befestigbar ist.

11. Steriles Abdecksystem nach Anspruch 9 oder 10, **dadurch gekennzeichnet, dass** eine zweite Platte (42) mit Durchgangsloch an der Drape (16) angeordnet ist, wobei das Durchgangsloch der zweiten Platte (42) die zweite Öffnung (47) bildet.

12. Steriles Abdecksystem nach Anspruch 11, **dadurch gekennzeichnet, dass** der Folienschlauch (33) mit dem ersten Ende (34) mit der zweiten Platte (42) mechanisch oder stoffschlüssig verbindbar ist.

13. Steriles Abdecksystem nach einem der Ansprüche 5 bis 12, **dadurch gekennzeichnet, dass** die erste und/oder die zweite Platte (32,42) an dem Anschlusselement (11) fixierbar ist, vorzugsweise mechanisch, magnetisch und/oder stoffschlüssig.

14. Steriles Abdecksystem nach einem der Ansprüche 5 bis 13, **dadurch gekennzeichnet, dass** die erste und/oder die zweite Platte (32,42) ein Klemmelement (50) zur Befestigung des jeweiligen Endes (34,38) des Folienschlauches (33) aufweist, wobei das Klemmelement (50) vorzugsweise plattenförmig ausgebildet ist.

15. Steriles Abdecksystem nach Anspruch 14, **dadurch gekennzeichnet, dass** ein Klemmelement (50) jeweils über eine Schnapp- oder Schraubverbindung mit der ersten oder zweiten Platte (32,42) zum Erzeugen einer Klemmkraft verbindbar ist.

16. Verfahren zum sterilen Verkleiden eines medizintechnischen Roboterarms mit einem eine Aufnahmebohrung (12) mit Innenwandung (14) aufweisenden Anschlusselement (11) für endoskopische Geräte mit folgenden Schritten:
a) Umhüllen des Roboterarms (10) mit einer sackförmigen Drape (16),
b) Einführen eines Folienschlauches (33) in eine erste Öffnung (37) in der sackförmigen Drape (16) und in die Aufnahmebohrung (12) mit Hilfe eines an einem ersten Ende (34) des Folienschlauches (33) befestigten Führungselementes (36),
c) Durchführen des Folienschlauches (33) durch die Aufnahmebohrung (12) mit Hilfe des Führungselementes (36) derart, dass die im Ausgangszustand des Folienschlauches (33) nach innen gewandte Oberfläche (35) des Folienschlauches im durch die Aufnahmebohrung (12) durchgeführten Zustand des Folienschlauches (33) der Innenwandung (14) der Aufnahmebohrung (12) zugewandt ist,
d) Durchführen des Folienschlauches (33) durch eine zweite Öffnung (47) in der sackförmigen Drape (16),
e) Lösen des Führungselementes (36) von dem ersten Ende (34) des Folienschlauches (33),
f) Befestigen des ersten Endes (34) des Folienschlauches (33) an der sackförmigen Drape (16) im Bereich der zweiten Öffnung (47).

17. Verfahren nach Anspruch 16, **dadurch gekennzeichnet, dass** im Ausgangszustand der Drape (16) die erste Öffnung (37) in der sackförmigen Drape (16) angeordnet ist, wobei die erste Öffnung (37) an das Führungselement (36) und/oder die Aufnahmebohrung (12) angepasst ist.

18. Verfahren nach Anspruch 17, **dadurch gekennzeichnet, dass** im Ausgangszustand der Drape (16) der Folienschlauch (33) mit dem zweiten Ende (38) im Bereich der ersten Öffnung (37) der sackförmigen Drape (16) befestigt ist.

19. Verfahren nach einem der Ansprüche 16 bis 18, **dadurch gekennzeichnet, dass** im Ausgangszustand der Drape (16) die zweite Öffnung (47) in der sackförmigen Drape (16) angeordnet ist, wobei die zweite Öffnung (47) an das Führungselement (36) und/oder die Aufnahmebohrung (12) angepasst ist.

20. Verfahren nach Anspruch 16, **dadurch gekennzeichnet, dass** zwischen Schritt a) und b) folgender Zwischenschritt durchgeführt wird:
a1) Durchstoßen der sackförmigen Drape (16) mit Hilfe des Führungselementes (36) zum Bilden der ersten Öffnung (37) in der sackförmigen Drape (16).

21. Verfahren nach einem der Ansprüche 16 bis 19 oder 20 **dadurch gekennzeichnet, dass** zwischen Schritt c) und d) folgender Zwischenschritt durchgeführt wird:
c1) Durchstoßen der sackförmigen Drape (16) mit Hilfe des Führungselementes (36) zum Bilden der zweiten Öffnung (47) in der sackförmigen Drape (16).

22. Verfahren nach einem der Ansprüche 16, 17 oder 19 bis 21, **dadurch gekennzeichnet, dass** zwischen Schritt a) und b) folgender Zwischenschritt durchgeführt wird:
a2) Befestigen eines zweiten Endes (38) des Folienschlauches (33) an der sackförmigen Drape (16) im Bereich der ersten Öffnung (37).

## Claims

1. A sterile cover system (1) for sterilely covering a medical technical robot arm (10) comprising a connecting element (11) for endoscopic devices, said connecting element having a receiving bore (12) with an inner wall (14), said cover system comprising
a sack-shaped drape (16) for enclosing the connecting element (11),
**characterized by**
a film tube (33) adapted to the receiving bore (12) for covering the inner wall (14), and
a removable guiding element (36) attached to a first end (34) of the film tube (33), said guiding element being adapted to the receiving bore (12) and extending into the film tube (33) in an initial state of the sterile cover system,
the film tube (33) being adapted to be inserted into and guided through the receiving bore (12) by means of the guiding element (36) in such a manner that a surface (35) of the film tube (33), facing inward in an initial state of the sterile cover system, faces toward the inner wall (14) of the receiving bore (12) in a state in which the film tube (33) has been guided through the receiving bore (12).

2. The sterile cover system according to claim 1, **characterized in that** the guiding element (36) is rod-shaped, a free end of the guiding element (36) being preferably provided with a tip (40) preferably suited for piercing the drape.

3. The sterile cover system according to claim 1 or, 2, **characterized in that** the sack-shaped drape (16) comprises at least one first opening (37) having a surrounding edge (39) and being adapted to the guiding element (36) and/or the receiving bore (12), said opening allowing the guiding element (36) and the film tube (33) to be at least partly passed therethrough.

4. The sterile cover system according to claim 3, **characterized in that** the film tube (33) can be fastened or is fastened by a second end (35) thereof to the surrounding edge (39) of the first opening (37).

5. The sterile cover system according to claim 3 or 4, **characterized in that** a first plate (32) with a throughgoing opening formed therein is arranged on the drape, said throughgoing hole forming the first opening (37).

6. The sterile cover system according to claim 5, **characterized in that** the film tube (33) is by its second end (38) tightly connected to the first plate (32).

7. The sterile cover system according to claim 5, **characterized in that** the film tube (33) can be connected by its second end (38) to the first plate mechanically or by a bonding connection.

8. The sterile cover system according to any one of claims 1 to 7, **characterized in that** the film tube (33) is made of an elastic material, preferably silicone.

9. The sterile cover system according to any one of claims 3 to 8, **characterized in that** the sack-shaped drape (16) comprises at least one second opening (47) having a surrounding edge (49) and being adapted to the guiding element and/or the receiving bore, said opening allowing the guiding element (36) and the film tube (33) to be passed at least partly therethrough.

10. The sterile cover system according to claim 9, **characterized in that** the film tube (33) can be fastened by its first end (34) to the surrounding edge (49) of the second opening (47).

11. The sterile cover system according to claim 9 or 10, **characterized in that** a second plate (42) with a throughgoing opening is arranged on the drape (16), the throughgoing opening of said second plate (42) forming the second opening (47).

12. The sterile cover system according to claim 11, **characterized in that** the film tube (33) can be connected by its first end (34) to the second plate (42) mechanically or by a bonding connection.

13. The sterile cover system according to any one of claims 5 to 12, **characterized in that** the first and/or the second plate (32,42) can be fixed to the connecting element (11), preferably mechanically, magnetically or by material connection.

14. The sterile cover system according to any one of claims 5 to 13, **characterized in that** the first and/or the second plate (32,42) comprises a clamping element (50) for attachment of the respective end (34,38) of the film tube (33), said clamping element (50) being preferably plate-shaped.

15. The sterile cover system according to claim 14, **characterized in that** a clamping element (50) can be connected respectively via a snap- or screw-type connection with the first and/or second plate (32,42) for generating a clamping force.

16. A method for sterilely covering a medical technical robot arm comprising a connecting element (11) for endoscopic devices, said connecting element having a receiving bore (12) with an inner wall (14), said method comprising the following steps:
a) enclosing the robot arm (10) with a sack-shaped drape (16),
b) introducing a film tube (33) into a first opening (37) in the sack-shaped drape (16) and into the receiving bore (12) with the aid of a guiding element (36) attached to a first end (34) of the film tube (33),
c) passing the film tube (33) through the receiving bore (12) with the aid of the guiding element (36) in such a manner that that the surface (35) of the film tube facing inward in the initial state of the film tube (33) is facing toward the inner wall (14) of the receiving bore (12) in the state wherein the film tube (33) has been passed through the receiving bore (12),
d) passing the film tube (33) through a second opening (47) in the sack-shaped drape (16),
e) detaching the guiding element (36) from the first end (34) of the film tube (33),
f) fastening the first end (34) of the film tube (33) to the sack-shaped drape (16) in the region of the second opening (47).

17. The method according to claim 16, **characterized in that**, in the initial state of the drape (16), the first opening (37) is arranged in the sack-shaped drape (16), the first opening (37) being adapted to the guiding element (36) and/or the receiving bore (12).

18. The method according to claim 17, **characterized in that**, in the initial state of the drape (16), the film tube (33) is by its second end (38) fastened in the region of the first opening (37) of the sack-shaped drape (16).

19. The method according to any one of claim 16 to 18, **characterized in that**, in the initial state of the drape (16), the second opening (47) is arranged in the sack-shaped drape (16), the second opening (47) being adapted to the guiding element (36) and/or the receiving bore (12).

20. The method according to claim 16, **characterized in that** the following intermediate step is performed between said steps a) and b):
a1) perforating the sack-shaped drape (16) with the aid of the guiding element (36) for forming the first opening (37) in the sack-shaped drape (16).

21. The method according to any one of claims 16 to 19 or 20, **characterized in that** the following intermediate step is performed between said steps c) and d):
c1) perforating the sack-shaped drape (16) with the aid of the guiding element (36) for forming the second opening (47) in the sack-shaped drape (16).

22. The method according to any one of claims 16, 17 or 19 to 21, **characterized in that** the following intermediate step is performed between said steps a) and b):
a2) fastening a second end (38) of the film tube (33) to the sack-shaped drape (16) in the region of the first opening (37).

## Revendications

1. Système de recouvrement stérile (1) pour l'habillage stérile d'un bras de robot (10) utilisé en technique médicale, comprenant un élément de raccordement (11) pour appareils endoscopiques présentant un alésage de réception (12) pourvu d'une paroi intérieure (14), comprenant
un drap (16) en forme de sac, destiné à envelopper l'élément de raccordement (11),
**caractérisé par**
un film tubulaire (33) adapté à l'alésage de réception (12) et destiné au recouvrement de la paroi intérieure (14), et
un élément de guidage (36) amovible, fixé à une première extrémité (34) du film tubulaire (33), qui est adapté à l'alésage de réception (12) et fait saillie, à l'état initial du système de recouvrement stérile, à l'intérieur du film tubulaire (33),
le film tubulaire (33) pouvant être introduit, au moyen de l'élément de guidage (36), dans l'alésage de réception (12) et conduit à travers celui-ci de façon telle qu'une surface (35) du film tubulaire (33), tournée vers l'intérieur à l'état initial du système de recouvrement stérile, est orientée vers la paroi intérieure (14) de l'alésage de réception (12) lorsque le film tubulaire (33) a traversé l'alésage de réception (12).

2. Système de recouvrement stérile selon la revendication 1, **caractérisé en ce que** l'élément de guidage (36) est réalisé en forme de barre, une pointe (40), qui est de préférence appropriée pour percer le drap, étant disposée de préférence à une extrémité libre de l'élément de guidage (36).

3. Système de recouvrement stérile selon la revendication 1 ou 2, **caractérisé en ce que** le drap (16) en forme de sac présente au moins une première ouverture (37) à bord périphérique (39), adaptée à l'élément de guidage (36) et/ou à l'alésage de réception (12), à travers laquelle on peut faire passer, au moins en partie, l'élément de guidage (36) et le film tubulaire (33).

4. Système de recouvrement stérile selon la revendication 3, **caractérisé en ce que** le film tubulaire (33) peut être fixé ou est fixé par une seconde extrémité (35) sur le bord périphérique (39) de la première ouverture (37).

5. Système de recouvrement stérile selon la revendication 3 ou 4, **caractérisé en ce qu'**une première plaque (32) munie d'un trou de passage est disposée au niveau du drap, le trou de passage formant la première ouverture (37).

6. Système de recouvrement stérile selon la revendication 5, **caractérisé en ce que** le film tubulaire (33) est relié, par la seconde extrémité (38), de manière solidaire à la première plaque (32).

7. Système de recouvrement stérile selon la revendication 5, **caractérisé en ce que** le film tubulaire (33) peut être relié par la seconde extrémité (38), mécaniquement ou par liaison de matière, à la première plaque (32).

8. Système de recouvrement stérile selon l'une des revendications 1 à 7, **caractérisé en ce que** le film tubulaire (33) se compose d'un matériau élastique, de préférence de silicone.

9. Système de recouvrement stérile selon l'une des revendications 3 à 8, **caractérisé en ce que** le drap (16) en forme de sac présente au moins une seconde ouverture (47) à bord périphérique (49), adaptée à l'élément de guidage et/ou à l'alésage de réception, à travers laquelle on peut faire passer, au moins en partie, l'élément de guidage (36) et le film tubulaire (33).

10. Système de recouvrement stérile selon la revendication 9, **caractérisé en ce que** le film tubulaire (33) peut être fixé, par la première extrémité (34), sur le bord périphérique (49) de la seconde ouverture (47).

11. Système de recouvrement stérile selon la revendication 9 ou 10, **caractérisé en ce qu'**une seconde plaque (42) munie d'un trou de passage est disposée au niveau du drap, le trou de passage de la seconde plaque (42) formant la seconde ouverture (47).

12. Système de recouvrement stérile selon la revendication 11, **caractérisé en ce que** le film tubulaire (33) peut, par la première extrémité (34), être relié mécaniquement ou par liaison de matière à la seconde plaque (42).

13. Système de recouvrement stérile selon l'une des revendications 5 à 12, **caractérisé en ce que** la première et/ou la seconde plaque (32, 42) peut (peuvent) être fixée(s) sur l'élément de raccordement (11), de préférence de façon mécanique, magnétique et/ou par liaison de matière.

14. Système de recouvrement stérile selon l'une des revendications 5 à 13, **caractérisé en ce que** la première et/ou la seconde plaque (32, 42) présente(nt) un élément de serrage (50) destiné à fixer chaque extrémité (34, 38) du film tubulaire (33), l'élément de serrage (50) étant réalisé, de préférence, en forme de plaque.

15. Système de recouvrement stérile selon la revendication 14, **caractérisé en ce qu'**un élément de serrage (50) peut être relié respectivement par l'intermédiaire d'un raccord à enclenchement ou d'un raccord fileté à la première ou à la seconde plaque (32, 42) pour produire une force de serrage.

16. Procédé pour l'habillage stérile d'un bras de robot utilisé en technique médicale, comprenant un élément de raccordement (11) pour appareils endoscopiques, présentant un alésage de réception (12) à paroi intérieure (14), comprenant les étapes suivantes consistant à :
a) envelopper le bras de robot (10) au moyen d'un drap (16) en forme de sac,
b) introduire un film tubulaire (33) dans une première ouverture (37) du drap (16) en forme de sac et dans l'alésage de réception (12) à l'aide d'un élément de guidage (36) fixé à une première extrémité (34) du film tubulaire (33),
c) faire passer le film tubulaire (33) au travers de l'alésage de réception (12) à l'aide de l'élément de guidage (36) de façon telle que la surface (35) du film tubulaire (33), tournée vers l'intérieur à l'état initial du film tubulaire (33), est orientée vers la paroi intérieure (14) de l'alésage de réception (12) lorsque le film tubulaire (33) a traversé l'alésage de réception (12),
d) faire passer le film tubulaire (33) à travers une seconde ouverture (47) du drap (16) en forme de sac,
e) détacher l'élément de guidage (36) de la première extrémité (34) du film tubulaire (33),
f) fixer la première extrémité (34) du film tubulaire (33) sur le drap (16) en forme de sac dans la région de la seconde ouverture (47).

17. Procédé selon la revendication 16, **caractérisé en ce qu'**à l'état initial du drap (16), la première ouverture (37) est disposée dans le drap (16) en forme de sac, la première ouverture (37) étant adaptée à l'élément de guidage (36) et/ou à l'alésage de réception (12).

18. Procédé selon la revendication 17, **caractérisé en ce qu'**à l'état initial du drap (16), le film tubulaire (33) est fixé, par la seconde extrémité (38), dans la région de la première ouverture (37) du drap (16) en forme de sac.

19. Procédé selon l'une des revendications 16 à 18, caractérisé en qu'à l'état initial du drap (16), la seconde ouverture (47) est disposée dans le drap (16) en forme de sac, la seconde ouverture (47) étant adaptée à l'élément de guidage (36) et/ou à l'alésage de réception (12).

20. Procédé selon la revendication 16, **caractérisé en ce que** l'étape intermédiaire suivante est réalisée entre l'étape a) et l'étape b) :
a1) percer le drap (16) en forme de sac à l'aide de l'élément de guidage (36) pour former la première ouverture (37) dans le drap (16) en forme de sac.

21. Procédé selon l'une des revendications 16 à 19, **caractérisé en ce que** l'étape intermédiaire suivante est réalisée entre l'étape c) et l'étape d) :
c1) percer le drap (16) en forme de sac à l'aide de l'élément de guidage (36) pour former la seconde ouverture (47) dans le drap (16) en forme de sac.

22. Procédé selon l'une des revendications 16, 17 ou 19 à 21, **caractérisé en ce que** l'étape intermédiaire suivante est réalisée entre l'étape a) et l'étape b) :
a2) fixer une seconde extrémité (38) du film tubulaire (33) sur le drap (16) en forme de sac dans la région de la première ouverture (37).
